# EUROPEAN PATENT APPLICATION

(11) **EP 4 796 523 A1**
(43) Date of publication of application: **26.08.2026**
(21) Application number: 24804721.9
(22) Date of filing: 02.04.2024
(51) Int. Cl.: C07C 17/25, C07C 21/18

(54) **METHOD FOR CO-PRODUCING 3,3,3-TRIFLUOROPROPENE AND 2,3,3,3-TETRAFLUOROPROPENE**

(30) Priority: 17.10.2023 CN 202311340442
(71) Applicant: Zhejiang Quhua Fluor-Chemistry Co Ltd, Quzhou, Zhejiang 324004 (CN)
(72) Inventor: TONG, Chaoli, Quzhou, Zhejiang 324004 (CN); REN, Yawen, Quzhou, Zhejiang 324004 (CN); HONG, Jiangyong, Quzhou, Zhejiang 324004 (CN); ZHANG, Yan, Quzhou, Zhejiang 324000 (CN); YANG, Bo, Quzhou, Zhejiang 324000 (CN); ZHAO, Yang, Quzhou, Zhejiang 324000 (CN); YU, Huimei, Quzhou, Zhejiang 324004 (CN)
(74) Representative: Becker, Eberhard
(86) International application number: PCT/CN2024/085539
(87) International publication number: WO 2025/081726

(57) **Abstract**

The present invention discloses a method for co-producing 3,3,3-trifluoropropene and 2,3,3,3-tetrafluoropropene, introducing 1,1,1,3-tetrachloropropane, 1,1,1,2,3-pentachloropropane and hydrogen fluoride into a first reactor to have a reaction in the presence of a first catalyst to obtain a reaction product of the first reactor; distilling the reaction product of the first reactor to obtain a 3,3,3-trifluoropropene product and a column bottom product, mixing the column bottom product with the hydrogen fluoride, and feeding a resulting mixture into a second reactor to have a reaction in the presence of a second catalyst to obtain a reaction product of the second reactor; and separating hydrogen chloride from the reaction product of the second reactor, and then carrying out water washing, alkali washing and drying to obtain a 2,3,3,3-tetrafluoropropene product. The present invention has the advantages of simple process, high efficiency, high operation flexibility, small investment and low energy consumption.

## Description

### Technical Field

The present invention relates to a method for preparing a fluorine-containing olefin and, in particular, to a method for co-producing 3,3,3-trifluoropropylene and 2,3,3,3-tetrafluoropropylene.

### Background Art

On September 15, 2021, China officially announced its accession to the Kigali Amendment. The Kigali Amendment aims to control HFCs (third-generation refrigerants, mainly hydrofluorocarbons) worldwide, requiring A5 countries including China to freeze the production and consumption of HFCs in 2024, and start reducing HFCs in 2029 until 80% reduction by 2045. Therefore, it is urgent to develop and research environment-friendly and efficient low-GWP refrigeration technology.

The fourth-generation novel refrigerants mainly refer to fluorine-containing olefins (HFOs), which have the advantages of zero ODP and extremely low GWP. A representative product is 2,3,3,3-tetrafluoropropene (HFO-1234yf, also known as R1234yf). The HFO-1234yf which has a boiling point of -29°C, an ODP value of 0, a GWP value of 4, and an atmospheric life of 11 days can be used as a refrigerant to replace HFC-134a in automotive air-conditioning systems. There are three main processes for preparing HFO-1234yf with industrial prospects: 3,3,3-trifluoropropylene process, hexafluoropropylene process and 1,1,2,3-tetrachloropropylene (TCP) process. The 3,3,3-trifluoropropylene process has a long route, high three wastes, and high product cost; the 1,1,2,3-tetrachloropropylene process has fewer reaction steps and high raw material utilization; and the hexafluoropropylene process has a long preparation route and a low overall yield. Other preparation processes are derived from the intermediate raw materials of these three routes.

3,3,3-Trifluoropropene (HFO-1243zf, also known as R1243zf). R1243zf has a boiling point of -22°C, an ODP value of 0, a GWP value of 1, and an atmospheric life of 7 days. R1243zf is mainly used as a low-GWP refrigerant and a pharmaceutical intermediate for synthesis. Depending on different main starting materials, the synthesis processes of can be divided into 1,1,1,3-tetrachloropropane fluorination process, trichloropropene (1,1,1-trichloropropene, 1,1,3-trichloro-1-propene) fluorination process, trifluoromonochloromethane-ethylene addition process, and carbene reaction. The 1,1,1,3-tetrachloropropane fluorination process is currently the most common process in industrial production.

Because the industrial equipment for producing HFO-1234yf and HFO-1243zf requires a large equipment investment and high operating cost, while the general-purpose equipment for simultaneously producing HFO-1234yf and HFO-1243zf requires a small equipment investment and can be flexibly adjusted for the output of HFO-1234yf and HFO-1243zf, and has high equipment operation flexibility. Thus, the technology for simultaneously preparing HFO-1234yf and HFO-1243zf has become a research hotspot.

For example, CN115322071A discloses a method for co-producing trifluoropropylene and tetrafluoropropylene using 1,1,1,2,3-pentafluoropropane as a raw material. This method uses 1,1,1,2,3-pentafluoropropane as a raw material to react in a fixed bed reactor in the presence of a metal ion-modified Mg-Al composite metal oxide which serves as a catalyst. During the reaction, H₂ is introduced, the feed space velocity is 400-450 h⁻¹, the reaction temperature is 330-350 °C, and the reaction pressure is normal pressure. This invention provides a novel method for preparing a fluoroolefin, which realizes the simultaneous preparation of 3,3,3-trifluoropropene, 1,3,3,3-tetrafluoropropene and 2,3,3,3-tetrafluoropropene in a one-step reaction. However, its disadvantage lies in that the main product is tetrafluoropropene, the selectivity of trifluoropropene is low, the production flexibility is poor, and the addition of hydrogen increases the difficulty and cost of subsequent separation.

### SUMMARY OF THE INVENTION

In view of the disadvantages in the prior art, the present invention provides a method for co-producing HFO-1243zf and HFO-1234yf with simple process, high yield, good selectivity and low energy consumption.

In order to solve the described technical problems, a technical solution adopted by the present invention is: a method for co-producing 3,3,3-trifluoropropene and 2,3,3,3-tetrafluoropropene, including the following steps:
(a) introducing 1,1,1,3-tetrachloropropane (HCC-250fb), 1,1,1,2,3-pentachloropropane (HCC-240db) and hydrogen fluoride (HF) into a first reactor to have a reaction in the presence of a first catalyst to obtain a reaction product of the first reactor;
(b) introducing the reaction product of the first reactor into a first distillation column for separation to obtain a top component of the first distillation column and a bottom component of the first distillation column;
(c) introducing the top component of the first distillation column into a second distillation column for separation to obtain a 3,3,3-trifluoropropene product and hydrogen chloride (HCl); introducing the bottom component of the first distillation column and hydrogen fluoride into a second reactor to have a reaction in the presence of a second catalyst to obtain a reaction product of the second reactor;
(d) introducing the reaction product of the second reactor into a third distillation column for separation to obtain a top component of the third distillation column and a bottom component of the third distillation column;
(e) introducing the top component of the third distillation column into a fourth distillation column for separation to obtain hydrogen chloride and a bottom component of the fourth distillation column; and
(f) performing water washing, alkali washing, and drying on the bottom component of the fourth distillation column to obtain a 2,3,3,3-tetrafluoropropene product.

As a preferred embodiment of the present invention, the first catalyst is a supported metal catalyst, the supported metal catalyst is at least one of CrCl₃, SbCl₃, SnCl₄, TiCl₄, and SbCl₅ supported on a carrier, wherein the carrier is one of activated carbon, silica, aluminum oxide, and a molecular sieve.

As a preferred embodiment of the present invention, the first catalyst is one of CrCl₃/C, SbCl₃/SiO₂, SnCl₄/Al₂O₃, TiCl₄/molecular sieve, and SbCl₅/C.

As a preferred embodiment of the present invention, the second catalyst is a supported antimony-based or chromium-based catalyst, wherein the mass percentage of antimony or chromium is 5-25%.

As a preferred embodiment of the present invention, the second catalyst is one of SbCl₅/SiO₂, Cr₂O₃/Al₂O₃, CrCl₃/C, and SbCl₅/molecular sieve.

As a preferred embodiment of the present invention, in step (a), a molar ratio of the 1,1,1,3-tetrachloropropane to the 1,1,1,2,3-pentachloropropane is 0.5-2:1, a molar ratio of the hydrogen fluoride to the 1,1,1,2,3-pentachloropropane is 7.5-60:1, and the reaction is performed at a temperature of 50-280 °C and a pressure of 0.1-1.5 MPa.

As a preferred embodiment of the present invention, in step (c), the amount of HF used is 3 to 10 times the molar number of the 1,1,1,2,3-pentachloropropane in step (a), and the reaction is performed at a temperature of 100-350 °C and a pressure of 0.1-1.5 MPa.

As a preferred embodiment of the present invention, the first reactor and the second reactor are configured as liquid phase reactors or gas phase fixed bed reactors.

As a preferred embodiment of the present invention, the bottom component of the third distillation column obtained in step (d) is recycled to the first reactor to continue the reaction.

The present invention realizes the co-production of HFO-1243zf and HFO-1234yf by means of two reactors. The first reactor is mainly for fluorination reaction of 1,1,1,3-tetrachloropropane and 1,1,1,2,3-pentachloropropane, and main equations are as follows:
CCl₃CH₂CH₂Cl (HCC-250fb) + 3HF → CF₃CH=CH₂ (HFO-1243zf) + 4HCl
CCl₃CHClCH₂Cl (HCC-240db) + 3HF → CF₃CCl=CH₂ (HCFO-1233xf) + 4HCl
CCl₃CHClCH₂Cl (HCC-240db) + 2HF → CF₂ClCCl=CH₂ (HCFO-1232xf) + 3HCl
CF₃CH=CH₂ (HFO-1243zf) + HF → CF₃CHFCH₃ (HFC-254eb)
CF₃CCl=CH₂ (HCFC-1233xf) + HF → CF₃CFClCH₃ (HCFC-244bb)

The boiling points of some substances in the present invention are as follows:

| Substance | Boiling point/°C | Substance | Boiling point/°C |
|---|---|---|---|
| CF₃CH=CH₂ (HFO-1243zf) | -25 | CF₂ClCCl=CH₂ (HCFO-1232xf) | 61 |
| CF₃CCl=CH₂ (HCFO-1233xf) | 12 | CF₃CFClCH₃ (HCFC-244bb) | 23 |
| CF₃CF=CH₂ (HFO-1234yf) | -29 | CF₃CHFCH₃ (HFC-254eb) | -1 |
| CF₃CF₂CH₃ (HFC-245cb) | -18 | | |

In the present invention, the first reactor may be configured as a liquid phase or gas phase reactor, 1,1,1,3-tetrachloropropane, 1,1,1,2,3-pentachloropropane and hydrogen fluoride are introduced into the first reactor to obtain a mixture containing CF₃CH=CH₂ (HFO-1243zf), CF₃CCl=CH₂ (HCFO-1233xf), CF₂ClCCl=CH₂ (HCFO-1232xf), CF₃CHFCH₃ (HFC-254eb), CF₃CFClCH₃ (HFC-244bb) and the like. Based on the different boiling points of the substances, distillation is performed. The material coming out of the first reactor is introduced into the first distillation column for separation, and HFO-1243zf and HCl are separated from the top of the column, and then HFO-1243zf and HCl are separated by the second distillation column to obtain a target product HFO-1243zf. The material at the bottom of the first distillation column directly enters a second reaction process.

The second reactor of the present invention is mainly for the fluorination reaction of CF₃CCl=CH₂, and the main equation is as follows:
CF₃CCl=CH₂ (HCFO-1233xf) + HF → CF₃CF=CH₂ (HFO-1234yf) + HCl
CF₃CF=CH₂ (HFO-1234yf) + HF → CF₃CF₂CH₃ (HFC-245cb) + HCl

The material coming out of the bottom of the first distillation column and HF are introduced into the second reactor to obtain a mixture containing CF₃CF=CH₂(HFO-1234yf), CF₃CF₂CH₃(HFC-245cb), and HCl. Based on the different boiling points of the substances, distillation is performed. The material coming out of the second reactor is introduced into the third distillation column, and HFO-1234yf and HCl are separated from the top of the third distillation column, and the material at the bottom of the column is returned to the first reactor for cyclic reaction. After HFO-1234yf and HCl are separated by the fourth distillation column, HCl is separated from the top of the column, and a crude HFO-1234yf product is obtained at the bottom of the column and is subjected to water washing, alkali washing and drying which are conventional technical means in the art, thus obtaining a 2,3,3,3-tetrafluoropropylene product.

The main raw materials of the present invention are 1,1,1,3-tetrachloropropane and 1,1,1,2,3-pentachloropropane, and the 1,1,1,3-tetrachloropropane and 1,1,1,2,3-pentachloropropane may be fed into the first reactor separately or after being mixed. A mixture of 1,1,1,3-tetrachloropropane and 1,1,1,2,3-pentachloropropane may be prepared by physically mixing 1,1,1,3-tetrachloropropane and 1,1,1,2,3-pentachloropropane, or by liquid phase chlorination of 1,1,1,3-tetrachloropropane. For example, by controlling the conversion rate of 1,1,1,3-tetrachloropropane, mixtures of 1,1,1,3-tetrachloropropane and 1,1,1,2,3-pentachloropropane with different molar ratios may be obtained.

Compared with the prior art, the present invention has the following advantages:
1. high yield and good selectivity: the total conversion rate of the raw materials 1,1,1,3-tetrachloropropane and 1,1,1,2,3-pentachloropropane reaches 96.8% or above, and the total selectivity of the products 3,3,3-trifluoropropene and 2,3,3,3-tetrafluoropropene reaches 98.2% or above;
2. simple process and low cost: the present invention improves the reaction efficiency by optimizing parameters such as reaction process, catalyst and material ratios, reaction temperature and pressure; the reaction temperature and reaction pressure are relatively low, the reaction conditions are mild and easy to control, the production process is simplified significantly, and energy consumption is reduced; the raw materials 1,1,1,3-tetrachloropropane and 1,1,1,2,3-pentachloropropane are cheap and widely available, further reducing production cost;
3. environmentally friendly and less three wastes: according to the present invention, unreacted raw materials and intermediate products can be recycled into the reactors to continue the reaction, thereby significantly reducing the discharge of the three wastes;
4. small investment and high operation flexibility: HFO-1243zf and HFO-1234yf products can be co-produced simultaneously by one set of set of equipment, and the proportion of products can be flexibly adjusted according to market demands, thus significantly reducing equipment investment.

### BRIEF DESCRIPTION OF THE DRAWINGS

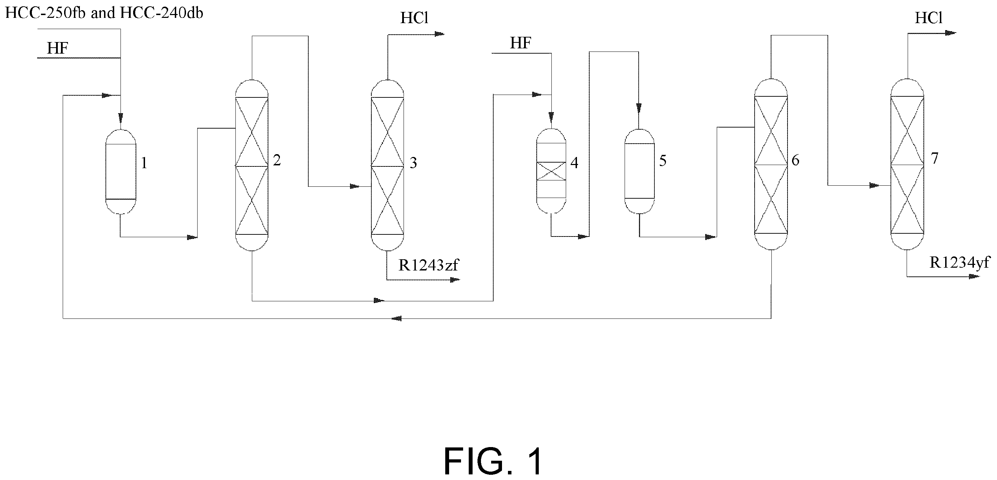

FIG. 1 is a process flow diagram of the present invention.

As shown in the figure: 1. first reactor; 2. first distillation column; 3. second distillation column; 4. mixer; 5. second reactor; 6. third distillation column; 7. fourth distillation column.

### DETAILED DESCRIPTION OF THE EMBODIMENTS

As shown in FIG. 1, a mixture of raw materials HCC-250fb and HCC-240db and HF are introduced into a first reactor 1 filled with a first catalyst to have a reaction, and a resulting reaction product is introduced into a first distillation column 2 to obtain a top component of the first distillation column and a bottom solution of the first distillation column that contains unreacted raw materials and other heavy components; the top component of the first distillation column is then introduced into a second distillation column 3 to obtain a 3,3,3-trifluoropropene product at the bottom of the column and hydrogen chloride at the top of the column; the bottom solution of the first distillation column and the hydrogen fluoride are introduced into a mixer 4 and mixed, and a resulting mixture then enters a second reactor 5 filled with a second catalyst to have a reaction in the presence of the second catalyst to obtain a reaction product of the second reactor; the reaction product of the second reactor is introduced into a third distillation column 6 for separation to obtain a top component of the third distillation column and a bottom component of the third distillation column; the top component of the third distillation column is then introduced into a fourth distillation column 7 for separation to obtain hydrogen chloride at the top of the column and a crude 2,3,3,3-tetrafluoropropene product at the bottom of the column; the crude 2,3,3,3-tetrafluoropropene product is then subjected to water washing, alkali washing and drying to obtain a 2,3,3,3-tetrafluoropropene product. The HCl separated from the top of the second distillation column 3 and from the top of the fourth distillation column 7 may be sent to other devices for use. The components at the bottom of the third distillation column may be returned to the first reactor 1 for recycling.

The technical solution of the invention is further described clearly and completely below in conjunction with embodiments. Apparently, embodiments described her are merely part, not all of embodiments of the present invention. All other embodiments obtained by a person of ordinary skill in the art based on the embodiments of the invention without creative efforts shall fall within the scope of the invention.

### Example 1

A mixture of raw materials HCC-250fb and HCC-240db and HF were introduced into a first reactor (liquid phase reactor) filled with a first catalyst CrCl₃/C to have a reaction at a set temperature and a set pressure in the presence of the first catalyst, and a resulting reaction product was then introduced into a first distillation column for separation; a bottom product of the first distillation column and the HF were mixed and introduced into a second reactor (liquid phase reactor) filled with a second catalyst SbCl₅/SiO₂ (the amount of Sb supported was 5wt%) to have a reaction at a set temperature and a set pressure in the presence of the second catalyst, and a resulting reaction product was then introduced into a third distillation column for separation. The reaction parameters of the first reactor and the second reactor are shown in Table 1, and the organic composition of outputs is shown in Table 2.

### Example 2

A mixture of raw materials HCC-250fb and HCC-240db and HF were introduced into a first reactor (gas phase fixed bed reactor) filled with a first catalyst SbCl₃/SiO₂ to have a reaction at a set temperature and a set pressure in the presence of the first catalyst, and a resulting reaction product was then introduced into a first distillation column for separation; a bottom product of the first distillation column and the HF were mixed and introduced into a second reactor (liquid phase reactor) filled with a second catalyst Cr₂O₃/Al₂O₃ (the amount of Cr supported was 10wt%) to have a reaction at a set temperature and a set pressure in the presence of the second catalyst, and a resulting reaction product was then introduced into a third distillation column for separation. The reaction parameters of the first reactor and the second reactor are shown in Table 1, and the organic composition of outputs is shown in Table 2.

### Example 3

A mixture of raw materials HCC-250fb and HCC-240db and HF were introduced into a first reactor (liquid phase reactor) filled with a first catalyst SnCl₄/Al₂O₃ to have a reaction at a set temperature and a set pressure in the presence of the first catalyst, and a resulting reaction product was then introduced into a first distillation column for separation; a bottom product of the first distillation column and the HF were mixed and introduced into a second reactor (gas phase fixed bed reactor) filled with a second catalyst CrCl₃/activated carbon (the amount of Cr supported was 15wt%) to have a reaction at a set temperature and a set pressure in the presence of the second catalyst, and a resulting reaction product was then introduced into a third distillation column for separation. The reaction parameters of the first reactor and the second reactor are shown in Table 1, and the organic composition of outputs is shown in Table 2.

### Example 4

A mixture of raw materials HCC-250fb and HCC-240db and HF were introduced into a first reactor (gas phase fixed bed reactor) filled with a first catalyst TiCl₄ to have a reaction at a set temperature and a set pressure in the presence of the first catalyst, and a resulting reaction product was then introduced into a first distillation column for separation; a bottom product of the first distillation column and the HF were mixed and introduced into a second reactor (gas phase fixed bed reactor) filled with a second catalyst SbCl₅/molecular sieve (the amount of Sb supported was 20wt%) to have a reaction at a set temperature and a set pressure in the presence of the second catalyst, and a resulting reaction product was then introduced into a third distillation column for separation. The reaction parameters of the first reactor and the second reactor are shown in Table 1, and the organic composition of outputs is shown in Table 2.

### Example 5

A mixture of raw materials HCC-250fb and HCC-240db and HF were introduced into a first reactor (gas phase fixed bed reactor) filled with a first catalyst SbCl₅ to have a reaction at a set temperature and a set pressure in the presence of the first catalyst, and a resulting reaction product was then introduced into a first distillation column for separation; a bottom product of the first distillation column and the HF were mixed and introduced into a second reactor (gas phase fixed bed reactor) filled with a second catalyst Cr₂O₃/Al₂O₃ (the amount of Cr supported was 25wt%) to have a reaction at a set temperature and a set pressure in the presence of the second catalyst, and a resulting reaction product was then introduced into a third distillation column for separation. The reaction parameters of the first reactor and the second reactor are shown in Table 1, and the organic composition of outputs is shown in Table 2.

**Table 1 Reaction parameters of Examples 1-5**

| Example | First reactor | | | Second reactor | | |
|---|---|---|---|---|---|---|
| | Temperature /°C | Pressure /MPa | *n*HCC-2S0fb:*n*HCC-240fb:*n*HF | Temperature /°C | Pressure /MPa | Molar ratio of HCC-240fb and hydrogen fluoride |
| 1 | 50 | 0.1 | 0.5:1:7.5 | 100 | 0.1 | 1:3 |
| 2 | 100 | 0.5 | 0.9:1:15 | 150 | 0.5 | 1:5 |
| 3 | 150 | 0.8 | 1.3:1:30 | 200 | 0.8 | 1:7 |
| 4 | 250 | 1.2 | 1.7:1:45 | 250 | 1.2 | 1:8 |
| 5 | 280 | 1.5 | 2.0:1:60 | 350 | 1.5 | 1:10 |

**Table 2 Reaction results of Examples 1-5**

| Example | Organic composition of output from the first reactor (molar ratio, %) | | | | | Organic composition of output from the second reactor (molar ratio, %) | | | |
|---|---|---|---|---|---|---|---|---|---|
| | R1243zf | R1233xf | HCC-250fb | HCC-240db | Others | R1234yf | HCC-250fb | HCC-240db | Others |
| 1 | 32.3 | 60.5 | 2.6 | 3.9 | 0.7 | 95.2 | 1.2 | 2.0 | 1.6 |
| 2 | 46.3 | *50.5* | 1.1 | 1.5 | 0.6 | 97.1 | 0.9 | 1.1 | 0.9 |
| 3 | 54.5 | 42.5 | 0.8 | 1.7 | 0.5 | 96.8 | 0.6 | 1.5 | 1.1 |
| 4 | 61.7 | 35.6 | 1.1 | 1.0 | 0.6 | 97.9 | 0.8 | 0.7 | 0.6 |
| 5 | 65.4 | 32.0 | 0.7 | 1.0 | 0.9 | 97.2 | 0.6 | 0.8 | 1.4 |

## Claims

1. A method for co-producing 3,3,3-trifluoropropene and 2,3,3,3-tetrafluoropropene, **characterized in that**, comprising the following steps:
(a) introducing 1,1,1,3-tetrachloropropane, 1,1,1,2,3-pentachloropropane and hydrogen fluoride into a first reactor to have a reaction in the presence of a first catalyst to obtain a reaction product of the first reactor;
(b) introducing the reaction product of the first reactor into a first distillation column for separation to obtain a top component of the first distillation column and a bottom component of the first distillation column;
(c) introducing the top component of the first distillation column into a second distillation column for separation to obtain a 3,3,3-trifluoropropene product and hydrogen chloride; introducing the bottom component of the first distillation column and the hydrogen fluoride into a second reactor to have a reaction in the presence of a second catalyst to obtain a reaction product of the second reactor;
(d) introducing the reaction product of the second reactor into a third distillation column for separation to obtain a top component of the third distillation column and a bottom component of the third distillation column;
(e) introducing the top component of the third distillation column into a fourth distillation column for separation to obtain hydrogen chloride and a bottom component of the fourth distillation column; and
(f) performing water washing, alkali washing, and drying on the bottom component of the fourth distillation column to obtain a 2,3,3,3-tetrafluoropropene product.

2. The method for co-producing 3,3,3-trifluoropropene and 2,3,3,3-tetrafluoropropene according to claim 1, **characterized in that**, the first catalyst is a supported metal catalyst, the supported metal catalyst is at least one of CrCl₃, SbCl₃, SnCl₄, TiCl₄, and SbCl₅ supported on a carrier, wherein the carrier is one of activated carbon, silica, aluminum oxide, and a molecular sieve.

3. The method for co-producing 3,3,3-trifluoropropene and 2,3,3,3-tetrafluoropropene according to claim 2, **characterized in that**, the first catalyst is one of CrCl₃/C, SbCl₃/SiO₂, SnCl₄/Al₂O₃, TiCl₄/molecular sieve, and SbCl₅/C.

4. The method for co-producing 3,3,3-trifluoropropene and 2,3,3,3-tetrafluoropropene according to claim 1, **characterized in that**, the second catalyst is a supported antimony-based or chromium-based catalyst, wherein a mass percentage of antimony or chromium is 5-25%.

5. The method for co-producing 3,3,3-trifluoropropene and 2,3,3,3-tetrafluoropropene according to claim 4, **characterized in that**, the second catalyst is one of SbCl₅/SiO₂, Cr₂O₃/Al₂O₃, CrCl₃/C, and SbCl₅/molecular sieve.

6. The method for co-producing 3,3,3-trifluoropropene and 2,3,3,3-tetrafluoropropene according to claim 1, **characterized in that**, in the step (a), a molar ratio of the 1,1,1,3-tetrachloropropane to the 1,1,1,2,3-pentachloropropane is 0.5-2: 1, a molar ratio of the hydrogen fluoride to the1,1,1,2,3-pentachloropropane is 7.5-60:1, and the reaction is performed at a temperature of 50-280°C and a pressure of 0.1-1.5 MPa.

7. The method for co-producing 3,3,3-trifluoropropene and 2,3,3,3-tetrafluoropropene according to claim 1, **characterized in that**, in the step (c), an amount of hydrogen fluoride used is 3 to 10 times a molar number of the 1,1,1,2,3-pentachloropropane in the step (a), and the reaction is performed at a temperature of 100-350°C and a pressure of 0.1-1.5 MPa.

8. The method for co-producing 3,3,3-trifluoropropene and 2,3,3,3-tetrafluoropropene according to claim 1, **characterized in that**, the first reactor and the second reactor are configured as liquid phase reactors or gas phase fixed bed reactors.

9. The method for co-producing 3,3,3-trifluoropropene and 2,3,3,3-tetrafluoropropene according to claim 1, **characterized in that**, the bottom component of the third distillation column obtained in the step (d) is recycled to the first reactor to continue the reaction.
